# EUROPEAN PATENT APPLICATION

(11) **EP 4 458 947 A1**
(43) Date of publication of application: **06.11.2024**
(21) Application number: 22916002.3
(22) Date of filing: 26.12.2022
(51) Int. Cl.: C12M 3/00, C07K 5/09, C07K 5/10, C07K 5/11, C07K 7/06, C07K 17/08, C08F 261/12, C08G 81/02, C08L 101/06, C12M 1/00, C12N 1/00, C12N 5/071

(54) **COATING SOLUTION FOR FORMING CELL SCAFFOLD AND PRODUCTION METHOD THEREOF**

(30) Priority: 27.12.2021 JP 2021212493; 06.01.2022 JP 2022001013; 06.01.2022 JP 2022001014; 10.05.2022 JP 2022077686
(71) Applicant: SEKISUI CHEMICAL CO., LTD., Osaka-shi Osaka 530-8565 (JP)
(72) Inventor: KOBAYASHI, Daigo, Mishima-gun, Osaka 618-0021 (JP); ARAI, Yuuhei, Mishima-gun, Osaka 618-0021 (JP)
(74) Representative: Ter Meer Steinmeister & Partner
(86) International application number: PCT/JP2022/047837
(87) International publication number: WO 2023/127779

(57) **Abstract**

Provided is a coating solution for forming a cell scaffold with which a cell scaffold having a large thickness can be easily formed and the proliferation of cells can be enhanced. A coating solution for forming a cell scaffold according to the present invention contains a peptide-conjugated resin having a synthetic resin moiety and a peptide moiety and an alcohol solvent, a content of the peptide-conjugated resin being 0.1 wt% or more.

## Description

### TECHNICAL FIELD

The present invention relates to a coating solution for forming a cell scaffold and a production method thereof.

### BACKGROUND ART

Cells of animals such as humans, mouse, rat, pig, cow, and monkey are used in research and development in academic fields, drug discovery fields, regenerative medicine fields, and the like. As a scaffold material used for culturing animal cells, adhesive proteins such as laminin and vitronectin, and natural polymer materials such as matrigel derived from mouse sarcoma are used.

A scaffold material using a synthetic resin and a scaffold material using a synthetic resin to which a peptide is bonded are also known. For example, Patent Documents 1 and 2 below disclose a scaffold material for cell culture, containing a peptide-conjugated polyvinyl alcohol derivative having a polyvinyl alcohol derivative moiety and a peptide moiety.

### Related Art Document

### Patent Documents

Patent Document 1: WO 2020/230884 A1
Patent Document 2: WO 2020/230885 A1

### SUMMARY OF THE INVENTION

### PROBLEMS TO BE SOLVED BY THE INVENTION

Cells can be cultured in a liquid medium using a scaffold material (cell scaffold), for example, processed or molded into a predetermined shape. Conventionally, a cell scaffold containing a synthetic resin to which a peptide is bonded is prepared by a solid phase method. However, in this method, the preparation process of a cell scaffold tends to be complicated.

The present inventors have found that when a cell scaffold having a large thickness is used, the proliferation of cells can be enhanced more than when a cell scaffold having a small thickness is used. However, in the solid phase method, the preparation process of a cell scaffold having a large thickness tends to be more complicated.

An object of the present invention is to provide a coating solution for forming a cell scaffold with which a cell scaffold having a large thickness can be easily formed and the proliferation of cells can be enhanced. Another object of the present invention is to provide a method for producing the coating solution for forming a cell scaffold.

### MEANS FOR SOLVING THE PROBLEMS

According to a broad aspect of the present invention, there is provided a coating solution for forming a cell scaffold (in the present specification, the "coating solution for forming a cell scaffold" may be abbreviated as "coating solution"), containing: peptide-conjugated resin having a synthetic resin moiety and a peptide moiety; and an alcohol solvent, a content of the peptide-conjugated resin being 0.1 wt% or more.

In a certain aspect of the coating solution according to the present invention, an amino acid content detected by a hydrolyzed amino acid composition analysis method is 75 µmol/L or more.

In a certain aspect of the coating solution according to the present invention, the synthetic resin moiety has a (meth)acrylic copolymer moiety or a polyvinyl alcohol derivative moiety.

In a certain aspect of the coating solution according to the present invention, the peptide-conjugated resin is a peptide-conjugated (meth)acrylic copolymer having a (meth)acrylic copolymer moiety and a peptide moiety, and the (meth)acrylic copolymer moiety has a structural unit derived from a (meth)acrylate compound (A) represented by the following Formula (A1) or the following Formula (A2).

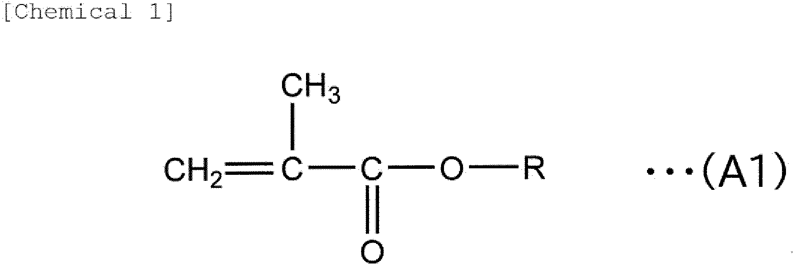

In the Formula (A1), R represents a hydrocarbon group having 2 or more and 18 or less carbon atoms.

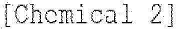

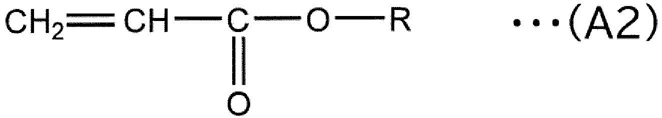

In the Formula (A2), R represents a hydrocarbon group having 2 or more and 18 or less carbon atoms.

In a certain aspect of the coating solution according to the present invention, the (meth)acrylic copolymer moiety has a structural unit derived from a (meth)acrylate compound (B) having a functional group capable of reacting with an amino group or a carboxyl group, and in the peptide-conjugated (meth)acrylic copolymer, the peptide moiety is bonded to the functional group capable of reacting with an amino group or a carboxyl group.

In a certain aspect of the coating solution according to the present invention, the peptide-conjugated resin is a peptide-conjugated polyvinyl alcohol derivative having a polyvinyl alcohol derivative moiety and a peptide moiety.

In a certain aspect of the coating solution according to the present invention, the peptide-conjugated polyvinyl alcohol derivative further has a structural unit derived from a (meth)acrylate compound (C) represented by the following Formula (C1) or the following Formula (C2).

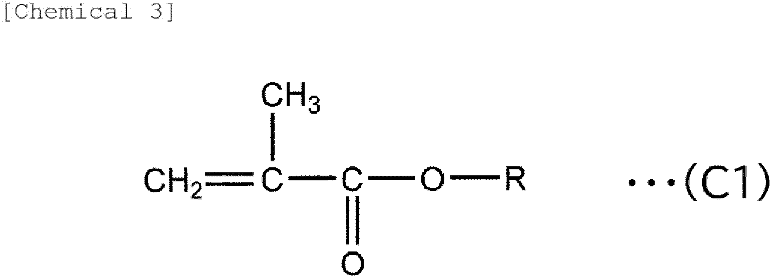

In the Formula (C1), R represents a hydrocarbon group having 2 or more and 18 or less carbon atoms.

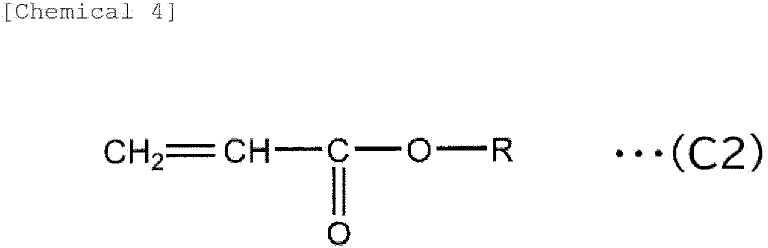

In the Formula (C2), R represents a hydrocarbon group having 2 or more and 18 or less carbon atoms.

In a certain aspect of the coating solution according to the present invention, the peptide-conjugated polyvinyl alcohol derivative further has a structural unit derived from a (meth)acrylate compound (D) having a functional group capable of reacting with an amino group or a carboxyl group, and
in the peptide-conjugated polyvinyl alcohol derivative, the peptide moiety is bonded to the functional group capable of reacting with an amino group or a carboxyl group.

In a certain aspect of the coating solution according to the present invention, the peptide-conjugated polyvinyl alcohol derivative is a peptide-conjugated polyvinyl acetal resin having a polyvinyl acetal moiety and a peptide moiety.

In a certain aspect of the coating solution according to the present invention, the peptide moiety has an RGD sequence.

According to a broad aspect of the present invention, there is provided a method for producing the above-described coating solution for forming a cell scaffold, including: a preparation step of preparing a solution containing a synthetic resin having a functional group capable of reacting with an amino group or a carboxyl group, a peptide, and a condensing agent; and a reaction step of reacting the synthetic resin with the peptide.

In a certain aspect of the method for producing the coating solution according to the present invention, the production method further includes a purification step.

In the present specification, the following method for producing a peptide-conjugated resin is also provided.

According to a broad aspect of the present invention, there is provided a method for producing a peptide-conjugated resin, including a step of preparing a first solution containing a first solvent, a synthetic resin having a functional group capable of reacting with an amino group or a carboxyl group, and a peptide; a step of preparing a second solution containing a second solvent and a condensing agent; and a step of mixing the first solution and the second solution.

In a certain aspect of the method for producing a peptide-conjugated resin according to the present invention, the production method further includes a reaction step of reacting the synthetic resin having a functional group capable of reacting with an amino group or a carboxyl group with the peptide.

In a certain aspect of the method for producing a peptide-conjugated resin according to the present invention, the production method further includes a purification step.

In a certain aspect of the method for producing a peptide-conjugated resin according to the present invention, the synthetic resin having a functional group capable of reacting with an amino group or a carboxyl group is a (meth)acrylic copolymer or a polyvinyl alcohol derivative.

In a certain aspect of the method for producing a peptide-conjugated resin according to the present invention, the peptide has an RGD sequence.

### EFFECT OF THE INVENTION

The coating solution for forming a cell scaffold according to the present invention contains a peptide-conjugated resin having a synthetic resin moiety and a peptide moiety and an alcohol solvent, a content of the peptide-conjugated resin being 0.1 wt% or more. Since the coating solution for forming a cell scaffold according to the present invention includes the above-described configuration, a cell scaffold having a large thickness can be easily formed and the proliferation of cells can be enhanced.

### MODES FOR CARRYING OUT THE INVENTION

Hereinafter, details of the present invention will be described.

### [Coating solution for forming cell scaffold]

The coating solution for forming a cell scaffold (hereinafter, may be abbreviated as "coating solution") according to the present invention contains a peptide-conjugated resin having a synthetic resin moiety and a peptide moiety and an alcohol solvent, a content of the peptide-conjugated resin being 0.1 wt% or more.

Since the coating solution according to the present invention includes the above-described configuration, a cell scaffold having a large thickness can be easily formed and the proliferation of cells can be enhanced. In the coating solution according to the present invention, the coating solution can be easily applied to a surface of a container or the like. Since the coating solution according to the present invention contains a relatively large amount of peptide-conjugated resin, it is possible to easily form a cell scaffold having a large thickness by drying the applied coating solution. In the coating solution according to the present invention, since a cell scaffold having a large thickness can be easily formed, the proliferation of cells can be enhanced.

In the coating solution according to the present invention, since it is not necessary to use a natural polymer material such as an extracellular matrix (ECM) as a material, it is inexpensive, has little variation between lots, and is excellent in safety.

### (Peptide-conjugated resin)

The coating solution contains a peptide-conjugated resin. The peptide-conjugated resin is a synthetic resin to which a peptide is bonded. The peptide-conjugated resin has a synthetic resin moiety and a peptide moiety. Only one kind of the peptide-conjugated resin may be used, or two or more kinds thereof may be used in combination.

From the viewpoint of exhibiting the effects of the present invention, the content of the peptide-conjugated resin in 100 wt% of the coating solution is 0.1 wt% or more.

The content of the peptide-conjugated resin in 100 wt% of the coating solution is preferably 0.3 wt% or more, more preferably 1 wt% or more, and still more preferably 3 wt% or more, and is preferably 15 wt% or less, more preferably 10 wt% or less, and still more preferably 8 wt% or less. When the content of the peptide-conjugated resin is the lower limit or more, a cell scaffold having a large thickness can be more easily formed, and the proliferation of cells can be further enhanced. When the content of the peptide-conjugated resin is the upper limit or less, the solubility of the peptide-conjugated resin in an alcohol solvent can be further enhanced, and coatability can be enhanced.

In the coating solution, the amino acid content detected by a hydrolyzed amino acid composition analysis method is preferably 75 µmol/L or more, more preferably 250 µmol/L or more, and still more preferably 750 µmol/L or more, and is preferably 10 mmol/L or less, more preferably 5 mmol/L or less, and still more preferably 3 mmol/L or less. The amino acid content corresponds to the amino acid content (µmol) detected in 1 L of the coating solution. When the amino acid content is the lower limit or more, the proliferation of cells can be further enhanced. When the amino acid content is the upper limit or less, the solubility of the peptide-conjugated resin in an alcohol solvent can be further enhanced, and coatability can be enhanced.

The amino acid content is measured, for example, as follows.

First, the coating solution is vacuum-dried, and the solvent is completely removed to obtain a dried solid. The obtained dried solid (5 mg) and 6 mol/L hydrochloric acid (500 µL) are placed in a vial container, degassed and sealed, and then heated at 110°C for 22 hours to hydrolyze the peptide. After the hydrolysis reaction, hydrochloric acid is removed by N2 purge. Then, 500 µL of a sodium citrate buffer solution at pH 2.2 is added and redissolved. The obtained solution is added to a centrifugal filter unit (Ultrafree-MC UFC30 series, pore size 0.1 µm, manufactured by Merck KGaA) and subjected to centrifugal filtration under the conditions of 13400 rpm and 3 min. The filtrate is analyzed by a post-column derivatization method using o-phthalaldehyde (hereinafter, may be referred to as "OPA") under the following analysis conditions and detection conditions. The filtrate is diluted 1 to 125 times with a sodium citrate buffer solution at pH 2.2 according to the spectral intensity and used for the measurement.

### <Analysis conditions>

- Column: Shim-pack Amino-Na (100 mmZ × 6.0 mmI.D.)
   Ammonia trap column: ISC-30/S0504 Na (50 mmL × 4.0 mmI.D.)
- Mobile phase: Amino acid mobile phase kit Na type (manufactured by SHIMADZU CORPORATION)
   Liquid A: Sodium citrate buffer solution
   Liquid B: Sodium citrate buffer solution
   Liquid C: Solution hydroxide solution
   Gradient elution (high separation mode)
- Flow rate: 0.4 mL/min
- Temperature: 60°C
- Injection volume: 10 µL

### <Detection conditions>

- Reaction reagent: Amino acid analysis kit OPA reagent (manufactured by SHIMADZU CORPORATION)
   Liquid A: Alkaline solution containing sodium hypochlorite
   Liquid B: Alkaline solution containing OPA and N-acetyl-L-cysteine
- Flow rate: 0.2 mL/min
- Temperature: 60°C
- Detection: Spectrofluorometric detector RF-20Axs
   Response: 1.5 sec
   Excitation wavelength: 350 nm
   Fluorescence wavelength: 450 nm
   Gain: × 1
   Sensitivity: Low

Next, a calibration curve is created using an amino acid mixture standard solution for automatic analysis of amino acids, type H (manufactured by Wako Pure Chemical Industries, Ltd., each amino acid concentration: 2.5 µmol/mL) as a standard reagent for quantification, and the obtained peak area value of each amino acid is quantified. The amino acid content (pmol/L) in the coating solution is calculated from the amount of the coating solution used for measurement, the obtained dried solid amount, and the amino acid concentration.

The synthetic resin moiety preferably has a (meth)acrylic copolymer moiety or a polyvinyl alcohol derivative moiety, and is more preferably a (meth)acrylic copolymer moiety or a polyvinyl alcohol derivative moiety. The peptide-conjugated resin is preferably a peptide-conjugated (meth)acrylic copolymer having a (meth)acrylic copolymer moiety and a peptide moiety or a peptide-conjugated polyvinyl alcohol derivative having a polyvinyl alcohol derivative moiety and a peptide moiety. In this case, the proliferation of cells can be further enhanced. The synthetic resin moiety may have both a (meth)acrylic copolymer moiety and a polyvinyl alcohol derivative moiety.

In the present specification, "(meth)acrylic" means one or both of "acrylic" and "methacrylic", and "(meth)acrylate" means one or both of "acrylate" and "methacrylate".

### <Peptide-conjugated (meth)acrylic copolymer>

From the viewpoint of further enhancing the proliferation of cells, the peptide-conjugated resin is preferably a peptide-conjugated (meth)acrylic copolymer having a (meth) acrylic copolymer moiety and a peptide moiety. The peptide-conjugated (meth)acrylic copolymer is a (meth)acrylic copolymer to which a peptide is bonded. Only one kind of the peptide-conjugated (meth)acrylic copolymer may be used, or two or more kinds thereof may be used in combination.

The (meth)acrylic copolymer moiety preferably has a structural unit derived from a (meth)acrylate compound (A) represented by the following Formula (A1) or the following Formula (A2). In this case, the hydrophobicity of the peptide-conjugated resin (peptide-conjugated (meth)acrylic copolymer) can be increased, and thus, the cell scaffold is less likely to be eluted into the liquid medium during cell culture, and the cell scaffold is less likely to be detached from the container or the like during cell culture. As a result, even when cells are cultured for a long period of time, the proliferation speed of the cells is less likely to decrease. The (meth)acrylate compound (A) may contain a (meth)acrylate compound represented by the following Formula (A1), may contain a (meth)acrylate compound represented by the following Formula (A2), or may contain both a (meth) acrylate compound represented by the following Formula (A1) and a (meth)acrylate compound represented by the following Formula (A2). When the (meth)acrylate compound (A) contains both a (meth)acrylate compound represented by the following Formula (A1) and a (meth) acrylate compound represented by the following Formula (A2), R in the following Formula (A1) and R in the following Formula (A2) may be the same or different. Only one kind of the (meth)acrylate compound (A) may be used, or two or more kinds thereof may be used in combination. Only one kind of each of the (meth)acrylate compound represented by the following Formula (A1) and the (meth)acrylate compound represented by the following Formula (A2) may be used, or two or more kinds thereof may be used in combination.

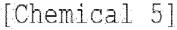

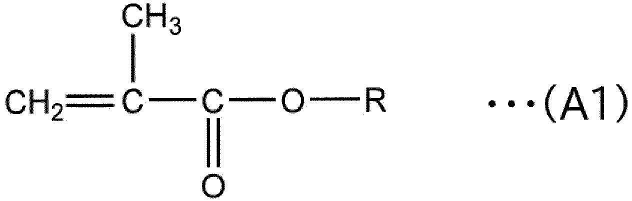

In the above Formula (A1), R represents a hydrocarbon group having 2 or more and 18 or less carbon atoms.

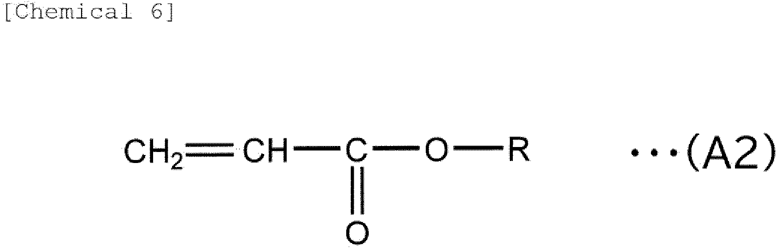

In the above Formula (A2), R represents a hydrocarbon group having 2 or more and 18 or less carbon atoms.

Each of R in the above Formula (A1) and R in the above Formula (A2) may be an aliphatic hydrocarbon group or an aromatic hydrocarbon group. From the viewpoint of improving the degree of solubility of the peptide-conjugated (meth)acrylic copolymer, each of R in the above Formula (A1) and R in the above Formula (A2) is preferably an aliphatic hydrocarbon group. The aliphatic hydrocarbon group may be linear, may have a branched structure, may have a double bond, or may not have a double bond. Each of R in the above Formula (A1) and R in the above Formula (A2) may be an alkyl group or an alkylene group.

The number of carbon atoms of R in the above Formula (A1) and the number of carbon atoms of R in the above Formula (A2) are each preferably 4 or more, more preferably 6 or more, still more preferably 8 or more, and particularly preferably 10 or more, and is preferably 16 or less, more preferably 14 or less, and most preferably 12. When the number of carbon atoms is the lower limit or more, the hydrophobicity of the peptide-conjugated (meth)acrylic copolymer can be increased, and thus, the cell scaffold is less likely to be eluted into the liquid medium during cell culture, and the cell scaffold is less likely to be detached from the container or the like during cell culture. When the number of carbon atoms is the upper limit or less, the solubility in an alcohol solvent can be further improved, and thus coatability and processability can be further improved. In particular, when the number of carbon atoms is 12, the effects of the present invention can be further more effectively exhibited, and coatability and processability can be further enhanced.

The content ratio of the structural unit derived from the (meth) acrylate compound (A) in 100 mol% of the total structural units of the (meth)acrylic copolymer moiety is preferably 25 mol% or more, more preferably 30 mol% or more, still more preferably 40 mol% or more, and particularly preferably 50 mol% or more, and is preferably 98 mol% or less, more preferably 95 mol% or less, still more preferably 90 mol% or less, further preferably 80 mol% or less, and particularly preferably 75 mol% or less. The content ratio of the structural unit derived from the (meth)acrylate compound (A) in 100 mol% of the total structural units of the (meth)acrylic copolymer moiety is preferably 25 mol% or more and 98 mol% or less, more preferably 30 mol% or more and 95 mol% or less, still more preferably 40 mol% or more and 90 mol% or less, further preferably 50 mol% or more and 80 mol% or less, and particularly preferably 50 mol% or more and 75 mol% or less. When the content ratio is the lower limit or more, the hydrophobicity of the peptide-conjugated (meth)acrylic copolymer can be increased, and thus, the cell scaffold is less likely to be eluted into the liquid medium during cell culture, and the cell scaffold is less likely to be detached from the container or the like during cell culture. When the content ratio is the upper limit or less, the solubility in an alcohol solvent can be further improved, and thus coatability and processability can be further improved.

The (meth)acrylic copolymer moiety preferably has a structural unit derived from a (meth)acrylate compound (B) having a functional group capable of reacting with an amino group or a carboxyl group. The (meth)acrylate compound (B) may have a functional group capable of reacting with an amino group, may have a functional group capable of reacting with a carboxyl group, and may have a functional group capable of reacting with an amino group and a functional group capable of reacting with a carboxyl group. Only one kind of the (meth) acrylate compound (B) may be used, or two or more kinds thereof may be used in combination.

Examples of the functional group capable of reacting with an amino group or a carboxyl group include a carboxyl group, a thiol group, an amino group, and a cyano group.

From the viewpoint of effectively exhibiting the effects of the present invention, in the peptide-conjugated (meth)acrylic copolymer, the peptide moiety is preferably bonded to the functional group capable of reacting with an amino group or a carboxyl group. More specifically, the carboxyl group or amino group of the amino acid constituting the peptide moiety is preferably bonded to the functional group capable of reacting with an amino group or a carboxyl group.

The functional group capable of reacting with an amino group or a carboxyl group is preferably a carboxyl group or an amino group. The (meth)acrylate compound (B) preferably has a carboxyl group or an amino group.

Examples of the (meth)acrylate compound (B) include (meth)acrylic acid, 3-butenoic acid, 4-pentenoic acid, 5-hexenoic acid, 6-heptenoic acid, 7-octenoic acid, benzene acrylic acid, (meth)acryloyloxyethylsuccinic acid, (meth)acryloyloxyethylphthalic acid, (meth)acryloyloxypropylsuccinic acid, (meth)acryloyloxypropylphthalic acid, (meth)acryloyloxyethylhexahydrosuccinic acid, (meth)acryloyloxyethylhexahydrophthalic acid, (meth)acryloyloxypropylhexahydrosuccinic acid, and (meth)acryloyloxypropylhexahydrophthalic acid.

The (meth)acrylate compound (B) is preferably (meth)acrylic acid, (meth)acryloyloxyethylsuccinic acid, (meth)acryloyloxypropylsuccinic acid, (meth)acryloyloxyethylhexahydrosuccinic acid, (meth)acryloyloxypropylhexahydrosuccinic acid, or butenoic acid, and more preferably (meth)acrylic acid. In this case, the effects of the present invention can be more effectively exhibited.

The content ratio of the structural unit derived from the (meth) acrylate compound (B) in 100 mol% of the total structural units of the (meth)acrylic copolymer moiety is preferably 2 mol% or more, more preferably 5 mol% or more, still more preferably 10 mol% or more, further preferably 20 mol% or more, and particularly preferably 25 mol% or more, and is preferably 75 mol% or less, more preferably 70 mol% or less, still more preferably 60 mol% or less, and particularly preferably 50 mol% or less. When the content ratio is the lower limit or more, the solubility in an alcohol solvent can be enhanced. When the content ratio is the upper limit or less, the culture stability of cells is easily maintained over an extended period of time.

The total content ratio of the structural unit derived from the (meth)acrylate compound (A) and the structural unit derived from the (meth)acrylate compound (B) in 100 mol% of the total structural units of the (meth)acrylic copolymer moiety is preferably 50 mol% or more, more preferably 65 mol% or more, still more preferably 80 mol% or more, still further preferably 90 mol% or more, particularly preferably 95 mol% or more, and most preferably 100 mol%. When the total content ratio is the lower limit or more, the effects of the present invention can be more effectively exhibited. The total content ratio may be 100 mol% or less or 90 mol% or less.

The (meth)acrylic copolymer moiety may include a structural unit derived from a (meth)acrylate compound different from both the (meth)acrylate compound (A) and the (meth)acrylate compound (B) as long as it is not contrary to the object of the present invention. The (meth)acrylic copolymer moiety may include a structural unit derived from a vinyl compound copolymerizable with the (meth)acrylate compound as long as it is not contrary to the object of the present invention.

The content ratio of the structural unit derived from the (meth)acrylate compound (A), the content ratio of the structural unit derived from the (meth)acrylate compound (B), and the content ratio of the structural unit derived from the (meth)acrylate compound (C) in the peptide-conjugated polyvinyl alcohol derivative described below in the (meth)acrylic copolymer moiety can be measured by, for example, nuclear magnetic resonance (NMR).

### <Peptide-conjugated polyvinyl alcohol derivative>

From the viewpoint of further enhancing the proliferation of cells, the peptide-conjugated resin is preferably a peptide-conjugated polyvinyl alcohol derivative having a polyvinyl alcohol derivative moiety and a peptide moiety. The polyvinyl alcohol derivative moiety is a moiety derived from a polyvinyl alcohol derivative. The polyvinyl alcohol derivative is a compound derived from polyvinyl alcohol. From the viewpoint of further enhancing the adhesiveness between the cell scaffold and the cell, the polyvinyl alcohol derivative is preferably a polyvinyl acetal resin, and the polyvinyl alcohol derivative moiety is preferably a polyvinyl acetal moiety. That is, the peptide-conjugated polyvinyl alcohol derivative is more preferably a peptide-conjugated polyvinyl acetal resin having a polyvinyl acetal moiety and a peptide moiety. Only one kind of each of the polyvinyl alcohol derivative and the polyvinyl acetal resin may be used, or two or more kinds thereof may be used in combination.

The polyvinyl alcohol derivative moiety and the polyvinyl acetal moiety preferably have an acetal group, a hydroxyl group, and an acetyl group in side chains. However, the polyvinyl alcohol derivative moiety and the polyvinyl acetal moiety may not have, for example, an acetyl group. For example, all of the acetyl groups of the polyvinyl alcohol derivative moiety and the polyvinyl acetal moiety are bonded to a linker, so that the polyvinyl alcohol derivative moiety and the polyvinyl acetal moiety may not have an acetyl group.

The polyvinyl acetal resin can be synthesized by acetalizing polyvinyl alcohol with an aldehyde.

The aldehyde used for acetalization of polyvinyl alcohol is not particularly limited. Examples of the aldehyde include an aldehyde having 1 to 10 carbon atoms. The aldehyde may or may not have a chain aliphatic group, a cyclic aliphatic group, or an aromatic group. The aldehyde may be a chain aldehyde or a cyclic aldehyde. Only one kind of the aldehyde may be used, or two or more kinds thereof may be used in combination.

From the viewpoint of further enhancing the adhesiveness between the cell scaffold and the cell, the aldehyde is preferably formaldehyde, acetaldehyde, propionaldehyde, butyraldehyde, or pentanal, and more preferably butyraldehyde. Therefore, the polyvinyl acetal resin is more preferably a polyvinyl butyral resin, and the polyvinyl acetal moiety is more preferably a polyvinyl butyral moiety. From the viewpoint of further enhancing the adhesiveness between the cell scaffold and the cell, the peptide-conjugated resin is preferably a peptide-conjugated polyvinyl butyral resin having a polyvinyl butyral moiety and a peptide moiety.

In the peptide-conjugated polyvinyl alcohol derivative, the acetalization degree of the polyvinyl alcohol derivative moiety and the polyvinyl acetal moiety (butyralization degree in the case of the polyvinyl butyral moiety) is preferably 40 mol% or more and more preferably 50 mol% or more, and is preferably 90 mol% or less and more preferably 85 mol% or less. When the acetalization degree is the lower limit or more, the fixability of cells can be further enhanced, and the cells efficiently proliferate. When the acetalization degree is the upper limit or less, solubility in a solvent can be improved.

In the peptide-conjugated polyvinyl alcohol derivative, the content ratio of the hydroxyl group (hydroxyl group amount) of the polyvinyl alcohol derivative moiety and the polyvinyl acetal moiety is preferably 15 mol% or more and more preferably 20 mol% or more, and is preferably 45 mol% or less, more preferably 30 mol% or less, and still more preferably 25 mol% or less.

In the peptide-conjugated polyvinyl alcohol derivative, the acetylation degree (acetyl group amount) of the polyvinyl alcohol derivative moiety and the polyvinyl acetal moiety is preferably 1 mol% or more and more preferably 2 mol% or more, and is preferably 5 mol% or less and more preferably 4 mol% or less. When the acetylation degree is the lower limit or more and the upper limit or less, the reaction efficiency between the polyvinyl acetal resin and a linker can be enhanced.

The acetalization degree, the acetylation degree, and the hydroxyl group amount of the polyvinyl alcohol derivative moiety and the polyvinyl acetal moiety can be measured by ¹H-NMR (nuclear magnetic resonance spectrum).

The peptide-conjugated polyvinyl alcohol derivative preferably further has a structural unit derived from a (meth)acrylate compound (C) represented by the following Formula (C1) or the following Formula (C2). In this case, formation of a hydrogen bond between peptides (particularly, hydrogen bond between molecules) can be suppressed, and thus, solubility in an alcohol solvent can be further improved. The (meth)acrylate compound (C) may contain a (meth)acrylate compound represented by the following Formula (C1), may contain a (meth)acrylate compound represented by the following Formula (C2), or may contain both a (meth)acrylate compound represented by the following Formula (C1) and a (meth)acrylate compound represented by the following Formula (C2). When the (meth)acrylate compound (C) contains both a (meth)acrylate compound represented by the following Formula (C1) and a (meth)acrylate compound represented by the following Formula (C2), R in the following Formula (C1) and R in the following Formula (C2) may be the same or different. Only one kind of the (meth)acrylate compound (C) may be used, or two or more kinds thereof may be used in combination. Only one kind of each of the (meth)acrylate compound represented by the following Formula (C1) and the (meth)acrylate compound represented by the following Formula (C2) may be used, or two or more kinds thereof may be used in combination.

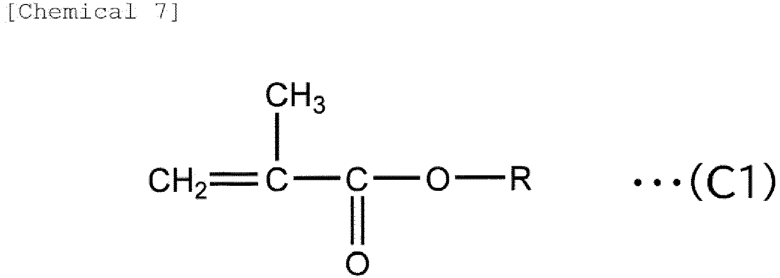

In the above Formula (C1), R represents a hydrocarbon group having 2 or more and 18 or less carbon atoms.

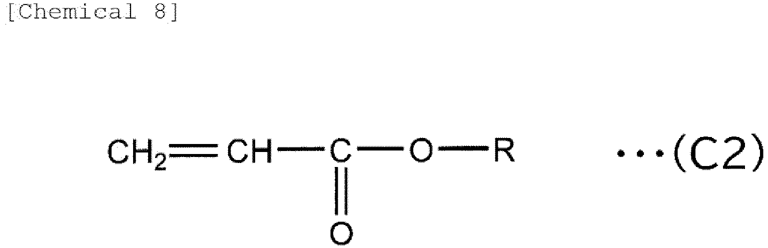

In the above Formula (C2), R represents a hydrocarbon group having 2 or more and 18 or less carbon atoms.

Each of R in the above Formula (C1) and R in the above Formula (C2) may be an aliphatic hydrocarbon group or an aromatic hydrocarbon group. From the viewpoint of improving the solubility of the peptide-conjugated polyvinyl alcohol derivative in an alcohol solvent, each of R in the above Formula (C1) and R in the above Formula (C2) is preferably an aliphatic hydrocarbon group. In this case, the aliphatic hydrocarbon group may be linear, may have a branched structure, may have a double bond, or may not have a double bond. Each of R in the above Formula (C1) and R in the above Formula (C2) may be an alkyl group or an alkylene group.

The number of carbon atoms of R in the above Formula (C1) and the number of carbon atoms of R in the above Formula (C2) are each preferably 4 or more, more preferably 6 or more, still more preferably 8 or more, and particularly preferably 10 or more, and is preferably 16 or less, more preferably 14 or less, and most preferably 12. When the number of carbon atoms is the lower limit or more and the upper limit or less, formation of a hydrogen bond between peptides can be suppressed, and thus, solubility in an alcohol solvent can be further improved.

In the peptide-conjugated polyvinyl alcohol derivative, the content ratio of the structural unit derived from the (meth)acrylate compound (C) is preferably 5 mol% or more and more preferably 10 mol% or more, and is preferably 50 mol% or less and more preferably 30 mol% or less. When the content ratio is the lower limit or more and the upper limit or less, formation of a hydrogen bond between peptides can be suppressed, and thus, solubility in an alcohol solvent can be further improved.

The peptide-conjugated polyvinyl alcohol derivative preferably further has a structural unit derived from a (meth)acrylate compound (D) having a functional group capable of reacting with an amino group or a carboxyl group. The (meth)acrylate compound (D) may have a functional group capable of reacting with an amino group, may have a functional group capable of reacting with a carboxyl group, and may have a functional group capable of reacting with an amino group and a functional group capable of reacting with a carboxyl group. Only one kind of the (meth)acrylate compound (D) may be used, or two or more kinds thereof may be used in combination.

Examples of the functional group capable of reacting with an amino group or a carboxyl group include a carboxyl group, a thiol group, an amino group, a hydroxyl group, and a cyano group.

From the viewpoint of effectively exhibiting the effects of the present invention, in the peptide-conjugated polyvinyl alcohol derivative, the peptide moiety is preferably bonded to the functional group capable of reacting with an amino group or a carboxyl group. More specifically, the carboxyl group or amino group of the amino acid constituting the peptide moiety is preferably bonded to the functional group capable of reacting with an amino group or a carboxyl group.

The functional group capable of reacting with an amino group or a carboxyl group is preferably a carboxyl group or an amino group. The (meth)acrylate compound (D) preferably has a carboxyl group or an amino group.

Examples of the (meth)acrylate compound (D) include the compounds listed as the (meth)acrylate compound (B).

The (meth)acrylate compound (D) is preferably (meth)acrylic acid, (meth)acryloyloxyethylsuccinic acid, (meth)acryloyloxypropylsuccinic acid, (meth)acryloyloxyethylhexahydrosuccinic acid, (meth)acryloyloxypropylhexahydrosuccinic acid, or butenoic acid, and more preferably (meth)acrylic acid. In this case, the effects of the present invention can be more effectively exhibited.

### <Peptide moiety>

The peptide moiety is a structural moiety derived from a peptide. The peptide moiety has an amino acid sequence. The peptide constituting the peptide moiety may be an oligopeptide or a polypeptide. Only one kind of the peptide may be used, or two or more kinds thereof may be used in combination.

The number of amino acid residues in the peptide moiety is preferably 3 or more, more preferably 4 or more, and still more preferably 5 or more, and is preferably 10 or less, more preferably 8 or less, and still more preferably 6 or less. When the number of amino acid residues is the lower limit or more and the upper limit or less, the adhesion to cells after seeding can be further enhanced, and the proliferation rate of cells can be further enhanced. However, the number of amino acid residues in the peptide moiety may be more than 10 or more than 15.

The peptide moiety preferably has a cell adhesive amino acid sequence. The cell adhesive amino acid sequence refers to an amino acid sequence whose cell adhesion activity has been confirmed by a phage display method, a sepharose bead method, or a plate coating method. As the phage display method, for example, the method described in "The Journal of Cell Biology, Volume 130, Number 5, September 1995 1189-1196" can be used. As the sepharose bead method, for example, the method described in "PROTEIN, NUCLEIC ACID, AND ENZYME, Vol. 45, No. 15 (2000) 2477" can be used. As the plate coating method, for example, the method described in "PROTEIN, NUCLEIC ACID, AND ENZYME, Vol. 45, No. 15 (2000) 2477" can be used.

Examples of the cell adhesive amino acid sequence include an RGD sequence (Arg-Gly-Asp), a YIGSR sequence (Tyr-Ile-Gly-Ser-Arg), a PDSGR sequence (Pro-Asp-Ser-Gly-Arg), an HAV sequence (His-Ala-Val), an ADT sequence (Ala-Asp-Thr), a QAV sequence (Gln-Ala-Val), an LDV sequence (Leu-Asp-Val), an IDS sequence (Ile-Asp-Ser), an REDV sequence (Arg-Glu-Asp-Val), an IDAPS sequence (Ile-Asp-Ala-Pro-Ser), a KQAGDV sequence (Lys-Gln-Ala-Gly-Asp-Val), and a TDE sequence (Thr-Asp-Glu). Examples of the cell adhesive amino acid sequence include the sequences described in "Pathophysiology, Vol. 9, No. 7, p. 527 to 535, 1990" and "Osaka Medical Center and Research Institute for Maternal and Child Health magazine, Vol. 8, No. 1, p. 58 to 66, 1992". The peptide moiety may have only one type of the cell adhesive amino acid sequence or two or more types thereof.

The cell adhesive amino acid sequence preferably has at least one of the above-described cell adhesive amino acid sequences, more preferably has at least an RGD sequence, a YIGSR sequence, or a PDSGR sequence, still more preferably has an RGD sequence, and particularly preferably has at least an RGD sequence represented by the following Formula (1). In this case, the adhesion to cells after seeding can be further enhanced, and the proliferation rate of cells can be further enhanced.

Arg-Gly-Asp-X Formula (1)

In the above Formula (1), X represents Gly, Ala, Val, Ser, Thr, Phe, Met, Pro, or Asn.

The peptide moiety may be linear or may have a cyclic peptide skeleton. The cyclic peptide skeleton is a cyclic skeleton composed of a plurality of amino acids. From the viewpoint of further effectively exhibiting the effects of the present invention, the cyclic peptide skeleton is preferably composed of four or more amino acids, more preferably composed of five or more amino acids, and preferably composed of ten or less amino acids.

In the peptide-conjugated resin, the content ratio of the peptide moiety is preferably 0.5 mol% or more, more preferably 1 mol% or more, still more preferably 2 mol% or more, and particularly preferably 5 mol% or more, and is preferably 25 mol% or less, more preferably 20 mol% or less, still more preferably 15 mol% or less, and particularly preferably 10 mol% or less. When the content ratio of the peptide moiety is the lower limit or more, the adhesion to cells after seeding can be further enhanced, and the proliferation rate of cells can be further enhanced. When the content ratio of the peptide moiety is the upper limit or less, the production cost can be suppressed. The content ratio (mol%) of the peptide moiety is the amount of substance of the peptide moiety with respect to the total of the amount of substance of structural units constituting the peptide-conjugated resin.

The content ratio of the peptide moiety can be measured by, for example, nuclear magnetic resonance (NMR).

### <Other details of peptide-conjugated resin>

The number average molecular weight of the peptide-conjugated (meth)acrylic copolymer is preferably 5000 or more, more preferably 10000 or more, and still more preferably 50000 or more, and is preferably 5000000 or less, more preferably 2500000 or less, and still more preferably 1000000 or less. When the number average molecular weight is the lower limit or more, the hydrophobicity of the peptide-conjugated (meth)acrylic copolymer can be increased, and thus, the cell scaffold is less likely to be eluted into the liquid medium during cell culture, and the cell scaffold is less likely to be detached from the container or the like during cell culture. When the number average molecular weight is the upper limit or less, the solubility in an alcohol solvent can be enhanced.

The number average molecular weight of the peptide-conjugated polyvinyl alcohol derivative is preferably 10000 or more, more preferably 50000 or more, and still more preferably 100000 or more, and is preferably 5000000 or less, more preferably 2500000 or less, and still more preferably 1000000 or less. When the number average molecular weight is the lower limit or more, the cell scaffold is less likely to be eluted into the liquid medium during cell culture, and the cell scaffold is less likely to be detached from the container or the like during cell culture. When the number average molecular weight is the upper limit or less, the solubility in an alcohol solvent can be enhanced.

The number average molecular weight of the peptide-conjugated resin (peptide-conjugated (meth)acrylic copolymer, peptide-conjugated polyvinyl alcohol derivative) can be measured by, for example, the following method. The peptide-conjugated resin is dissolved in tetrahydrofuran (THF) to prepare a 0.2 wt% solution of the peptide-conjugated resin. Next, evaluation is performed under the following measurement conditions using a gel permeation chromatography (GPC) measuring device (APC system, manufactured by Waters Corporation).

Column: HSPgel HR MB-M 6.0 × 150 mm
Flow rate: 0.5 mL/min
Column temperature: 40°C
Injection volume: 10 µL
Detector: RI, PDA
Standard sample: Polystyrene

### (Alcohol solvent)

The coating solution contains an alcohol solvent. Only one kind of the alcohol solvent may be used, or two or more kinds thereof may be used in combination.

Examples of the alcohol solvent include methanol, ethanol, butanol, isobutanol, sec-butanol, tert-butanol, isopropanol, propanol, benzyl alcohol, octyl alcohol, lauryl alcohol, and ethylene glycol.

From the viewpoint of favorably forming a resin film (cell scaffold) having a uniform thickness, the alcohol solvent is preferably a lower alcohol having 2 or more and 4 or less carbon atoms, more preferably ethanol, propanol, or butanol, and still more preferably butanol.

The content of the alcohol solvent in 100 wt% of the coating solution is preferably 80 wt% or more, more preferably 85 wt° or more, and still more preferably 90 wt% or more, and is preferably 99.9 wt% or less, more preferably 99 wt% or less, and still more preferably 98 wt% or less. When the content of the alcohol solvent is the lower limit or more and the upper limit or less, the solubility of the peptide-conjugated resin in an alcohol solvent can be further enhanced, and coatability can be enhanced.

The total content of the peptide-conjugated resin and the alcohol solvent in 100 wt% of the coating solution is preferably 80 wt% or more, more preferably 85 wt% or more, and still more preferably 90 wt% or more. The total content may be 100 wt% or less or less than 100 wt%.

### (pH adjusting agent)

The coating solution preferably contains a pH adjusting agent. By using the pH adjusting agent, the solubility of the peptide-conjugated resin in an alcohol solvent can be adjusted. Examples of the pH adjusting agent include an organic acid, an inorganic acid, an organic base, and an inorganic base. Examples of the pH adjusting agent include acetic acid, trifluoroacetic acid, hydrochloric acid, triethylamine, and N,N-diisopropylethylamine. Only one kind of the pH adjusting agent may be used, or two or more kinds thereof may be used in combination.

The content of the pH adjusting agent is preferably 0.1 parts by weight or more and more preferably 0.5 parts by weight or more, and is preferably 20 parts by weight or less and more preferably 15 parts by weight or less, with respect to 100 parts by weight of the alcohol solvent.

### (Other components)

The coating solution may contain other components different from the above-described components (the peptide-conjugated resin, the alcohol solvent, and the pH adjusting agent) within a range in which the effects of the present invention are not inhibited. Examples of the other components include solvents other than alcohol solvents, and polysaccharides. Only one kind of the other components may be used, or two or more kinds thereof may be used in combination.

Examples of the solvent other than the alcohol solvents include water, dimethyl sulfoxide, N,N-dimethylformamide, N-methylpyrrolidone, dichloromethane, and acetonitrile.

It is preferable that the coating solution does not substantially contain animal-derived raw materials. Since the coating solution does not contain animal-derived raw materials, it is possible to provide a cell scaffold having high safety and little variation in quality during production. The phrase "does not substantially contain animal-derived raw materials" means that the animal-derived raw materials in the coating solution are 3 wt% or less. In the coating solution, the animal-derived raw materials in the coating solution are preferably 1 wt% or less and most preferably 0 wt%. That is, it is most preferable that the coating solution does not contain any animal-derived raw materials.

### (Method for producing coating solution)

The method for producing a coating solution according to the present invention is a method for producing the above-described coating solution. The method for producing the coating solution according to the present invention includes a preparation step of preparing a solution containing a synthetic resin having a functional group capable of reacting with an amino group or a carboxyl group, a peptide, and a condensing agent, and a reaction step of reacting the synthetic resin with the peptide.

In the present specification, the "synthetic resin having a functional group capable of reacting with an amino group or a carboxyl group" may be referred to as "synthetic resin X".

Therefore, the method for producing the coating solution according to the present invention includes a preparation step of preparing a solution containing a synthetic resin X, a peptide, and a condensing agent, and a reaction step of reacting the synthetic resin X with the peptide.

The synthetic resin X may have a functional group capable of reacting with an amino group, may have a functional group capable of reacting with a carboxyl group, and may have a functional group capable of reacting with an amino group and a functional group capable of reacting with a carboxyl group.

Examples of the synthetic resin X include a (meth)acrylate copolymer and a polyvinyl acetal derivative. More specific examples of the synthetic resin X include a (meth)acrylate copolymer obtained by polymerizing a monomer mixture containing the (meth)acrylate compound (A) and the (meth)acrylate compound (B), and a polyvinyl acetal derivative having a structural unit derived from the (meth)acrylate compound (D). Only one kind of the synthetic resin X may be used, or two or more kinds thereof may be used in combination.

Examples of the peptide include peptides having the number of amino acid residues, the amino acid sequence, and the like described in the section of the peptide moiety. Only one kind of the peptide may be used, or two or more kinds thereof may be used in combination.

As the condensing agent, a conventionally known condensing agent can be used. Examples of the condensing agent include 1-ethyl-3-(3-dimethylaminopropyl)carbodiimide hydrochloride, N,N'-dicyclohexylcarbodiimide, 1-hydroxybenzotriazole, 1-hydroxy-7-azabenzotriazole, diphenylphosphoric azide, hexamethylphosphoric triamide, O-(benzotriazole-1-yl)-N,N,N',N'-tetramethyluronium hexafluorophosphate, 2-chloro-4,6-dimethoxy-1,3,5,-triazine, 4-(4,6-dimethoxy-1,3,5,-triazine-2-yl)-4-methylmorpholinium chloride, 4,6-dimethoxy-1,3,5,-triazine-2-yl)-(2-octoxy-2-oxoethyl)dimethylammonium trifluoromethanesulfonate, 2,2,6,6,-tetramethylpiperidine, and (1-cyano-2-ethoxy-2-oxoethylideneaminooxy)dimethylaminomorpholinocarbenium hexafluorophosphate. Only one kind of the condensing agent may be used, or two or more kinds thereof may be used in combination.

In the preparation step, a solution containing a synthetic resin X, a peptide, a condensing agent, and a solvent is prepared. The solution containing a synthetic resin X, a peptide, a condensing agent, and a solvent may be a dispersion. In the preparation step, the solution may be obtained by mixing a synthetic resin X, a peptide, and a condensing agent in the same solvent, and for example, the solution may be obtained by mixing a solution A containing at least one component among a synthetic resin X, a peptide, and a condensing agent with a solution B containing at least one component among a synthetic resin X, a peptide, and a condensing agent.

The preparation step preferably includes a step of preparing a first solution containing a first solvent, a synthetic resin X, and a peptide, a step of preparing a second solution containing a second solvent and a condensing agent, and a step of mixing the first solution and the second solution. In this case, the control of the condensation reaction can be facilitated. The first solvent and the second solvent may be the same kind of solvent or different kinds of solvents. The second solution may be prepared after the first solution is prepared, or the first solution may be prepared after the second solution is prepared. The first solution and the second solution may be prepared simultaneously.

Each of the solvent, the first solvent, and the second solvent may be an alcohol solvent or a solvent other than the alcohol solvent. When the method for producing a coating solution does not include a purification step described below, each of the solvent, the first solvent, and the second solvent is preferably an alcohol solvent.

Examples of the solvent, the first solvent, and the second solvent include methanol, ethanol, butanol, dimethylformamide (DMF), tetrahydrofuran (THF), acetonitrile, acetone, methyl ethyl ketone (MEK), dimethyl sulfoxide (DMSO), and water.

In the reaction step, the synthetic resin X and the peptide are reacted. It is preferable to bond the functional group capable of reacting with an amino group or a carboxyl group of the synthetic resin X to the carboxyl group or amino group of the amino acid constituting the peptide. Thereby, the peptide-conjugated resin can be obtained. When an alcohol solvent is used as the solvent, a liquid containing a peptide-conjugated resin and an alcohol solvent can be obtained in the reaction step. The reaction conditions in the reaction step are not particularly limited.

The method for producing a coating solution preferably further includes a purification step. The purification method in the purification step is not particularly limited, and examples thereof include (1) a method in which a liquid phase containing a peptide-conjugated resin is recovered after liquid-liquid phase separation, (2) a method in which a peptide-conjugated resin is recovered after reprecipitation, and (3) a method in which ion exchange is performed using an ion exchange resin. When the method for producing a coating solution includes the purification step, a coating solution with less impurities can be obtained by removing an unreacted condensing agent or peptide. In the purification step, a plurality of purification methods may be combined. The solution obtained after purification may be used as it is as a coating solution, or a solution obtained by volatilizing a solvent and then redissolving the solvent in an alcohol solvent may be used as a coating solution.

### (Other details of coating solution)

A cell scaffold can be formed by applying the coating solution to a surface of a container or the like and drying the applied coating solution. Examples of the coating method include a spin coating method, a gravure coating method, a flexo coating method, a spray coating method, a cast coating method, a dip coating method, a jet dispensing method, and an inkjet method.

The thickness of the cell scaffold is not particularly limited. The average thickness of the cell scaffold is preferably 10 nm or more, more preferably 20 nm or more, and still more preferably 30 nm or more, and is preferably 1 µm or less, more preferably 500 nm or less, and still more preferably 200 nm or less. When the average thickness is the lower limit or more, the proliferation of cells can be enhanced. In the coating solution according to the present invention, a cell scaffold having an average thickness of the lower limit or more can be easily formed. When the average thickness is the upper limit or less, it is easy to obtain a cell scaffold having less surface irregularities and good appearance. The average thickness of the cell scaffold may be 50 nm or more or 100 nm or more, and may be 1000 µm or less or 500 µm or less.

A surface roughness Ra of the cell scaffold is preferably 10 nm or less and more preferably 5 nm or less. When the surface roughness Ra of the cell scaffold is the upper limit or less, it is possible to obtain a cell scaffold with little variation in proliferation of cells. The surface roughness Ra of the cell scaffold can be adjusted to the upper limit or less by appropriately selecting a coating method of the coating solution, drying conditions, and the like.

The cell scaffold is used as a scaffold for cells when the cells are cultured.

Examples of the cells include cells of animals such as humans, mouse, rat, pig, cow, and monkey. Examples of the cells include somatic cells, and examples thereof include stem cells, progenitor cells, and mature cells. The somatic cells may be cancer cells.

Examples of the stem cells include somatic stem cells and embryonic stem cells, and examples thereof include neural stem cells, hematopoietic stem cells, mesenchymal stem cells (MSC), iPS cells, ES cells, Muse cells, embryonic cancer cells, embryonic germ stem cells, and mGS cells.

Examples of the mature cells include nerve cells, cardiomyocytes, retinal cells, and hepatocytes.

The cell scaffold is preferably used for two-dimensional culture (plane culture), three-dimensional culture, or suspension culture of cells, more preferably used for two-dimensional culture (plane culture) or three-dimensional culture, and still more preferably used for two-dimensional culture.

The cell scaffold is preferably used for serum-free medium culture. Since the cell scaffold contains the peptide-conjugated resin, the adhesiveness of cells can be enhanced even in a serum-free medium culture containing no feeder cell or adhesive protein, and in particular, initial fixation rate after cell seeding can be enhanced.

### (Substrate for cell culture)

A substrate for cell culture can be prepared by applying the coating solution to a surface of a substrate and drying the applied coating solution. The substrate for cell culture includes a substrate and the cell scaffold formed on the surface of the substrate. In the substrate for cell culture, the cell scaffold is a dried product layer of the coating solution described above. In the substrate for cell culture, the cell scaffold is preferably a resin film.

The shape and size of the substrate are not particularly limited. As the substrate, for example, a fiber, a nonwoven fabric, a hollow fiber, a particle, a film, a porous membrane, or the like can be used.

As the fiber, for example, fibers having an average length of 1 µm to 10 mm and an average diameter of 100 nm to 300 µm can be used. Examples of the material of the fiber include a synthetic resin, cellulose, and glass. Examples of the synthetic resin include a styrene copolymer, polyester, polyamide, a (meth)acrylic resin, and polyvinyl alcohol.

As the nonwoven fabric, for example, a nonwoven fabric having an average fiber diameter of 0.01 µm to 10 µm, an apparent density of 1 kg/m³ to 100 kg/m³, and an average thickness of 10 µm to 1000 µm can be used. Examples of the material of the nonwoven fabric include a synthetic resin and cellulose. Examples of the synthetic resin include polypropylene, polyester, polyamide, and polyphenylene sulfide.

As the hollow fiber, for example, a hollow fiber having an inner diameter of 50 µm to 1000 µm, a membrane thickness of 10 µm to 400 µm, and a pore size of 0.001 µm to 0.5 µm can be used. Examples of the material of the hollow fiber include polyethylene, polyamide, polyester, polyethersulfone, polysulfone, polyvinylidene fluoride, polytetrafluoroethylene, polypropylene, cellulose, a polyacrylonitrile copolymer, polyvinyl chloride, polyethylene vinyl alcohol, and ceramic.

As the particle, for example, particles having an average particle size of 10 µm to 1000 µm and a specific gravity of 0.5 to 5.0 can be used. Examples of the material of the particle include a synthetic resin, polysaccharide, and silica. Examples of the synthetic resin include polystyrene, a (meth)acrylic resin, and divinylbenzene.

As the film, for example, a film having a thickness of 1 µm to 1000 µm can be used. The film may be a single layer or a multilayer. Examples of the material of the film include polyethylene, polypropylene, an ethylene-vinyl acetate copolymer, polyester, polyamide, a silicone-based elastomer, a polystyrene-based elastomer, and an ionomer resin.

As the porous membrane, for example, a porous membrane having an average pore size of 0.01 µm to 10 µm, a porosity of 1 vol% to 95 vol%, and an average membrane thickness of 1 µm to 100 µm can be used. Examples of the material of the porous membrane include polyester, polyethylene, polyimide, and a fluororesin.

As the method for applying the coating solution to the surface of the substrate and coating conditions, an optimum method and optimum conditions can be appropriately selected according to the size, shape, and the like of the substrate.

For example, in the case of a film-shaped substrate, the substrate can be coated with the coating solution using a roll coating method. Examples of the roll coating method include a gravure coating method, a flexo coating method, a reverse coating method, a slot die coating method, a lip coating method, and a knife coating method. The coating solution may be applied to the entire surface of the film-shaped substrate, or the coating solution may be applied so that a pattern such as a dot pattern or a stripe pattern is formed.

For example, in the case of a porous substrate or a fibrous substrate, the substrate can be coated with the coating solution using a dip coating method.

### (Cell culture container)

A cell culture container can be prepared by applying the coating solution to a surface of a container body and drying the applied coating solution. The cell culture container includes a container body and the cell scaffold formed on the surface of the container body. In the cell culture container, the cell scaffold is a dried product layer of the coating solution described above. In the cell culture container, the cell scaffold is preferably a resin film.

As the container body, a conventionally known container body (container) can be used. The shape and size of the container body are not particularly limited. As the container body, for example, a 2 to 384-well plate, a single-layer flask, a multi-layer flask, a multi-plane flask, a dish, a roller bottle, a bag, an insert cup, a microchannel chip, or the like can be used.

Examples of the material of the container body include synthetic resins, metals, and glass. Examples of the synthetic resin include polystyrene, polyethylene, polypropylene, polyethersulfone, polycarbonate, polyester, polyisoprene, a cycloolefin polymer, polyimide, polyamide, polyamideimide, a (meth)acrylic resin, an epoxy resin, and silicone.

As the method for applying the coating solution to the surface of the container body and coating conditions, an optimum method and optimum conditions can be appropriately selected according to the size, shape, and the like of the container body.

For example, the coating solution can be applied to the surface of the container body using a cast coating method, a spray coating method, a spin coating method, or the like. The coating solution may be applied to the surface of the container body so that a pattern such as a dot pattern or a stripe pattern is formed. As a method for applying the coating solution so as to form a pattern, for example, an inkjet method, a screen printing method, a micro contact printing method, or the like can be used.

### (Method for producing peptide-conjugated resin)

The present specification also discloses a method for producing a peptide-conjugated resin including the following configuration.

A method for producing a peptide-conjugated resin, including (1) a step of preparing a first solution containing a first solvent, a synthetic resin (synthetic resin X) having a functional group capable of reacting with an amino group or a carboxyl group, and a peptide; (2) a step of preparing a second solution containing a second solvent and a condensing agent; and (3) a step of mixing the first solution and the second solution.

The method for producing a peptide-conjugated resin is a method for producing a peptide-conjugated resin having a synthetic resin moiety and a peptide moiety. According to the method for producing a peptide-conjugated resin, as described above, a coating solution can be suitably produced. The peptide-conjugated resin obtained by the method for producing a peptide-conjugated resin is preferably used as a coating solution containing the peptide-conjugated resin and an alcohol solvent, but may not be used as a coating solution.

In the method for producing a peptide-conjugated resin, the second solution may be prepared after the first solution is prepared, or the first solution may be prepared after the second solution is prepared. The first solution and the second solution may be prepared simultaneously.

The details of the synthetic resin X (synthetic resin having a functional group capable of reacting with an amino group or a carboxyl group) in the method for producing a peptide-conjugated resin are the same as the details of the synthetic resin X described in the section of the method for producing a coating solution described above.

The synthetic resin X in the method for producing a peptide-conjugated resin is preferably a (meth)acrylate copolymer or a polyvinyl acetal derivative. The synthetic resin X in the method for producing a peptide-conjugated resin is more preferably a (meth)acrylate copolymer obtained by polymerizing a monomer mixture containing the (meth)acrylate compound (A) and the (meth)acrylate compound (B), or a polyvinyl acetal derivative having a structural unit derived from the (meth)acrylate compound (D). Only one kind of the synthetic resin X may be used, or two or more kinds thereof may be used in combination.

The details of the peptide in the method for producing a peptide-conjugated resin are the same as the details of the peptide described in the section of the method for producing a coating solution described above. That is, examples of the peptide include peptides having the number of amino acid residues, the amino acid sequence, and the like described in the section of the peptide moiety. Only one kind of the peptide may be used, or two or more kinds thereof may be used in combination.

The number of amino acid residues in the peptide is preferably 3 or more, more preferably 4 or more, and still more preferably 5 or more, and is preferably 10 or less, more preferably 8 or less, and still more preferably 6 or less. When the number of amino acid residues is the lower limit or more and the upper limit or less, the adhesion to cells after seeding can be further enhanced, and the proliferation rate of cells can be further enhanced. However, the number of amino acid residues in the peptide may be more than 10 or more than 15.

The peptide preferably has a cell adhesive amino acid sequence, more preferably has at least an RGD sequence, a YIGSR sequence, or a PDSGR sequence, still more preferably has an RGD sequence, and particularly preferably has at least an RGD sequence represented by the above Formula (1).

The peptide may be linear or may have a cyclic peptide skeleton. The cyclic peptide skeleton is a cyclic skeleton composed of a plurality of amino acids. The cyclic peptide skeleton is preferably composed of four or more amino acids, more preferably composed of five or more amino acids, and preferably composed of ten or less amino acids.

The details of the condensing agent in the method for producing a peptide-conjugated resin are the same as the details of the condensing agent described in the section of the method for producing a coating solution described above. Only one kind of the condensing agent may be used, or two or more kinds thereof may be used in combination.

The details of the first solvent and the second solvent in the method for producing a peptide-conjugated resin are the same as the details of the first solvent and the second solvent described in the section of the method for producing a coating solution described above. Only one kind of each of the first solvent and the second solvent may be used, or two or more kinds thereof may be used in combination.

In the method for producing a peptide-conjugated resin, the first solvent is preferably an alcohol solvent, and the second solvent is preferably an alcohol solvent.

The method for producing a peptide-conjugated resin preferably further includes a reaction step of reacting the synthetic resin X with the peptide.

In the reaction step, the synthetic resin X and the peptide are reacted. It is preferable to bond the functional group capable of reacting with an amino group or a carboxyl group of the synthetic resin X to the carboxyl group or amino group of the amino acid constituting the peptide. Thereby, the peptide-conjugated resin can be obtained. When an alcohol solvent is used as the solvent, a liquid containing a peptide-conjugated resin and an alcohol solvent can be obtained in the reaction step. The reaction conditions in the reaction step are not particularly limited.

The method for producing a peptide-conjugated resin preferably further includes a purification step. The purification method in the purification step is not particularly limited, and examples thereof include (1) a method in which a liquid phase containing a peptide-conjugated resin is recovered after liquid-liquid phase separation, (2) a method in which a peptide-conjugated resin is recovered after reprecipitation, and (3) a method in which ion exchange is performed using an ion exchange resin. When the method for producing a peptide-conjugated resin includes the purification step, a peptide-conjugated resin with less impurities can be obtained by removing an unreacted condensing agent or peptide. In the purification step, a plurality of purification methods may be combined.

Hereinafter, the present invention will be described in more detail with reference to Examples and Comparative Examples. The present invention is not limited only to these Examples.

The content ratio of the structural unit and the content ratio of the peptide moiety in the obtained peptide-conjugated resin were measured by 1H-NMR (nuclear magnetic resonance spectrum) after dissolving the peptide-conjugated resin in DMSO-d6 (dimethyl sulfoxide).

The following peptides (A) and (B) were prepared.

### Peptide (A):

Linear peptide having an amino acid sequence of Gly-Arg-Gly-Asp-Ser (five amino acid residues, described as GRGDS in the table)

### Peptide (B):

Cyclic peptide having an amino acid sequence of Arg-Gly-Asp-Phe-Lys (five amino acid residues, forming a cyclic skeleton by bonding Arg and Lys, Phe is a D form, described as c-RGDfK in the table)

### (Example 1)

### <Preparation of coating solution>

### Preparation of synthetic resin (synthetic resin X) having functional group capable of reacting with amino group or carboxyl group:

As a polyvinyl acetal resin, a polyvinyl butyral resin having an acetalization degree (butyralization degree) of 65 mol%, a hydroxyl group amount of 32 mol%, and an acetyl group amount of 3 mol% was prepared. Acrylic acid (30 parts by weight) and a polyvinyl acetal resin (70 parts by weight) were dissolved in 255 parts by weight of tetrahydrofuran to obtain a polymer mixed solution. PERBUTYL O (0.015 parts by weight) (manufactured by NOF CORPORATION) was dissolved in the obtained polymer mixed solution, and the mixture was reacted at 90°C for 6 hours. Next, the solution after the reaction was mixed with 30000 parts by weight of water. The obtained precipitate was vacuum-dried at 80°C for 3 hours to prepare a polyvinyl acetal resin X having a structural unit derived from acrylic acid (polyvinyl butyral resin having a structural unit derived from acrylic acid) as the synthetic resin X.

### Preparation step:

DMF was prepared as the first solvent. DMF was prepared as the second solvent. The peptide (A) was prepared as the peptide. As a condensing agent, 1-ethyl-3-(3-dimethylaminopropyl)carbodiimide hydrochloride was prepared. The polyvinyl acetal resin X (50 parts by weight) and the peptide (2 parts by weight) were mixed with 1000 parts by weight of the first solvent to prepare a first solution. A condensing agent (1 part by weight) was mixed with 1000 parts by weight of the second solvent to prepare a second solution. The first solution and the second solution were mixed to prepare a solution containing the polyvinyl acetal resin X, the peptide, and the condensing agent.

### Reaction step:

The obtained solution was reacted at 40°C for 2 hours, and a carboxyl group in the structural unit derived from acrylic acid of the polyvinyl acetal resin X and an amino group of Gly of the peptide were dehydrated and condensed, thereby obtaining a solution containing a peptide-conjugated polyvinyl acetal resin (described as "Resin X1" in the table).

### Purification step:

The obtained solution containing a peptide-conjugated polyvinyl acetal resin was diluted 100 times with DMF, and added dropwise to a column packed with an ion exchange resin (manufactured by Organo Corporation) at a rate of 0.3 mL/min for washing. The solution after washing was vacuum-dried at 60°C for 3 hours to obtain a dried solid, the dried solid was dissolved in butanol (alcohol solvent), and then 5 parts by weight of acetic acid (pH adjusting agent) was added to 100 parts by weight of butanol (alcohol solvent) to obtain a coating solution containing a peptide-conjugated polyvinyl acetal resin (resin X1) and butanol (alcohol solvent). The content of the peptide-conjugated polyvinyl acetal resin in the coating solution was set to 0.1 wt%.

### <Preparation of cell culture container>

To each well of a 6-well plate, 20 pL of the obtained coating solution was applied by a cast coating method, and then the alcohol solvent was removed by vacuum drying at 60°C for 3 hours. In this way, a cell culture container in which a cell scaffold (resin film), which is a dried product layer of the coating solution, was disposed on the bottom surface of each well was obtained.

### (Examples 2 and 4 and Comparative Example 2)

Coating solutions and cell culture containers were obtained in the same manner as in Example 1, except that the content of the peptide-conjugated polyvinyl acetal resin in the coating solution was set to the content shown in Tables 3 and 4.

### (Examples 3 and 5)

The content ratio of the peptide moiety in the peptide-conjugated polyvinyl acetal resin was adjusted to the content ratio shown in Table 1 by changing the amount of the peptide contained in the first solution to 0.3 parts by weight (Example 3) and 4 parts by weight (Example 5), respectively. The content of the peptide-conjugated polyvinyl acetal resin in the coating solution was set to the content shown in Table 3. Coating solutions and cell culture containers were obtained in the same manner as in Example 1 except for these. In the table, the peptide-conjugated polyvinyl acetal resin used in Example 3 was described as "Resin X2", and the peptide-conjugated polyvinyl acetal resin used in Example 5 was described as "Resin X3".

### (Example 6)

The peptide (B) was prepared as the peptide, and the amount of the peptide contained in the first solution was set to 2 parts by weight. A carboxyl group in the structural unit derived from acrylic acid of the polyvinyl acetal resin X and an amino group of Lys of the peptide were dehydrated and condensed to synthesize a peptide-conjugated polyvinyl acetal resin (described as "Resin X4" in the table) . A coating solution and a cell culture container were obtained in the same manner as in Example 1, except that this peptide-conjugated polyvinyl acetal resin was used and the content of the peptide-conjugated polyvinyl acetal resin in the coating solution was set to the content shown in Table 4.

### (Example 7)

Acrylic acid (15 parts by weight), dodecyl acrylate (15 parts by weight), and a polyvinyl acetal resin (70 parts by weight) were dissolved in 255 parts by weight of tetrahydrofuran to obtain a polymer mixed solution. A polyvinyl acetal resin X having a structural unit derived from acrylic acid and a structural unit derived from dodecyl acrylate (polyvinyl butyral resin having a structural unit derived from acrylic acid and a structural unit derived from dodecyl acrylate) was prepared in the same manner as in Example 1 as the synthetic resin X except for this.

The peptide (B) was prepared as the peptide, and the amount of the peptide contained in the first solution was set to 4 parts by weight. A peptide-conjugated polyvinyl acetal resin (described as "Resin X5" in the table) was synthesized in the same manner as in Example 6 except for this. A coating solution and a cell culture container were obtained in the same manner as in Example 1, except that this peptide-conjugated polyvinyl acetal resin was used and the content of the peptide-conjugated polyvinyl acetal resin in the coating solution was set to the content shown in Table 4.

### (Example 8)

A coating solution and a cell culture container were obtained in the same manner as in Example 1, except that the alcohol solvent in the coating solution was changed to ethanol and the content of the peptide-conjugated polyvinyl acetal resin in the coating solution was set to the content shown in Table 4.

### (Comparative Example 1)

The polyvinyl acetal resin X (resin before reacting with the peptide, described as "Resin Y1" in the table) prepared in Example 1 was used. The alcohol solvent in the coating solution was changed to ethanol and the content of the resin Y1 in the coating solution was set to the content shown in Table 4. Coating solutions and cell culture containers were obtained in the same manner as in Example 1 except for these.

### (Example 9)

### <Preparation of coating solution>

### Preparation of synthetic resin (synthetic resin X) having functional group capable of reacting with amino group or carboxyl group:

Dodecyl acrylate (70 parts by weight) and acrylic acid (30 parts by weight) were dissolved in 27 parts by weight of tetrahydrofuran to obtain an acrylic monomer solution. In the obtained acrylic monomer solution, 0.0575 parts by weight of Irgacure 184 (manufactured by BASF) was dissolved, and the obtained solution was applied onto a PET film. A (meth)acrylic copolymer solution was obtained by irradiating the coated material with light having a wavelength of 365 nm at an integrated light quantity of 2000 mJ/cm² using a UV conveyor device ("ECS301G1" manufactured by Eye Graphics Co., Ltd.) at 25°C. The obtained (meth)acrylic copolymer solution was vacuum-dried at 80°C for 3 hours to obtain a (meth)acrylic copolymer. In this way, a (meth)acrylic copolymer X was prepared as the synthetic resin X.

### Preparation step:

DMF was prepared as the first solvent. DMF was prepared as the second solvent. The peptide (A) was prepared as the peptide. As a condensing agent, 1-ethyl-3-(3-dimethylaminopropyl)carbodiimide hydrochloride was prepared. The (meth)acrylic copolymer X (50 parts by weight) and the peptide (2 parts by weight) were mixed with 1000 parts by weight of the first solvent to prepare a first solution. A condensing agent (1 part by weight) was mixed with 1000 parts by weight of the second solvent to prepare a second solution. The first solution and the second solution were mixed to prepare a solution containing the (meth)acrylic copolymer X, the peptide, and the condensing agent.

### Reaction step:

The obtained solution was reacted at 40°C for 2 hours, and a carboxyl group in the structural unit derived from acrylic acid of the (meth)acrylic copolymer X and an amino group of Gly of the peptide were dehydrated and condensed to synthesize a peptide-conjugated (meth)acrylic copolymer (described as "Resin X6" in the table).

### Purification step:

The obtained solution containing a peptide-conjugated (meth)acrylic copolymer was diluted 100 times with DMF, and added dropwise to a column packed with an ion exchange resin (manufactured by Organo Corporation) at a rate of 0.3 mL/min for washing. The solution after washing was vacuum-dried at 60°C for 3 hours to obtain a dried solid, the dried solid was dissolved in butanol (alcohol solvent), and then 5 parts by weight of acetic acid (pH adjusting agent) was added to 100 parts by weight of butanol (alcohol solvent) to obtain a coating solution containing a peptide-conjugated (meth)acrylic copolymer (resin X6) and butanol (alcohol solvent). The content of the peptide-conjugated (meth)acrylic copolymer in the coating solution was set to 0.1 wt%.

### <Preparation of cell culture container>

To each well of a 6-well plate, 20 µL of the obtained coating solution was applied by a cast coating method, and then the alcohol solvent was removed by vacuum drying at 60°C for 3 hours. In this way, a cell culture container in which a cell scaffold (resin film), which is a dried product layer of the coating solution, was disposed on the bottom surface of each well was obtained.

### (Examples 10 and 11 and Comparative Example 3)

Coating solutions and cell culture containers were obtained in the same manner as in Example 9, except that the content of the peptide-conjugated (meth)acrylic copolymer in the coating solution was set to the content shown in Table 5.

### (Example 12)

The peptide (B) was prepared as the peptide, and the amount of the peptide contained in the first solution was set to 2 parts by weight. A carboxyl group in the structural unit derived from acrylic acid of the (meth)acrylic copolymer X and an amino group of Lys of the peptide were dehydrated and condensed to synthesize a peptide-conjugated (meth)acrylic copolymer (described as "Resin X7" in the table). A coating solution and a cell culture container were obtained in the same manner as in Example 9, except that this peptide-conjugated (meth)acrylic copolymer was used and the content of the peptide-conjugated (meth)acrylic copolymer in the coating solution was set to the content shown in Table 5.

### (Example 13)

A coating solution and a cell culture container were obtained in the same manner as in Example 9, except that 70 parts by weight of butyl acrylate was used instead of dodecyl acrylate and the content of the peptide-conjugated (meth)acrylic copolymer in the coating solution was set to the content shown in Table 5. The peptide-conjugated (meth)acrylic copolymer prepared in Example 13 was described as "Resin X8" in the table.

### (Example 14)

A coating solution and a cell culture container were obtained in the same manner as in Example 9, except that 70 parts by weight of octyl acrylate was used instead of dodecyl acrylate and the content of the peptide-conjugated (meth)acrylic copolymer in the coating solution was set to the content shown in Table 5. The peptide-conjugated (meth)acrylic copolymer prepared in Example 14 was described as "Resin X9" in the table.

### (Evaluation)

### (1) Solubility (maximum dissolution amount at 60°C) in ethanol

The solubility (maximum dissolution amount at 60°C) of each of the obtained resins X1 to X9 and Y1 in ethanol was evaluated according to the following criteria.

### <Criteria for determining solubility in ethanol>

○○: The maximum dissolution amount is 1 wt% or more.
○: The maximum dissolution amount is 0.5 wt% or more and less than 1 wt%.
Δ: The maximum dissolution amount is 0.1 wt% or more and less than 0.5 wt%.
×: The maximum dissolution amount is less than 0.1 wt%.

### (2) Amino acid content detected in coating solution

In the obtained coating solution, the amino acid content detected by a hydrolyzed amino acid composition analysis method was measured by the above-described method.

### (3) Average thickness of cell scaffold

In the obtained cell culture container, the average thickness of the cell scaffold (resin film) was measured using a reflection spectrometric film thickness meter ("OPTELICS" manufactured by Lasertec Corporation).

### (4) Culture evaluation of cells (proliferation of cells)

The following liquid medium was prepared.

### R-STEM (manufactured by ROHTO Pharmaceutical Co., Ltd.)

To the obtained cell culture container, 1 mL of phosphate buffered saline was added, the mixture was left to stand still in an incubator at 37°C for 1 hour, and then the phosphate buffered saline was removed from the cell culture container.

A cell suspension containing 5×10⁴ cells (human fat-derived mesenchymal stem cell manufactured by LONZA KK., Model No.: PT-5006) was prepared in 1.5 mL of the liquid medium. This cell suspension was seeded into each well of a 6-well plate. Next, the 6-well plate was shaken 5 times to the right and left, and placed in an incubator at 37°C and a CO₂ concentration of 5% to perform culture.

The number of cells after culture for 5 days was counted using NucleoCounter NC-3000 (manufactured by M&S TechnoSystems Inc.). The doubling time was calculated according to the following calculation method, and the proliferation of cells was determined according to the following criteria.

### <Method for calculating doubling time>

X = log2 ÷ {log (N (5))-log (N (0))} × T
X: Doubling time (hours)
N (0): Number of seeded cells (cells)
N (5): Cell number (cells) after 5 days from seeding
T: Culture time (hours)
T is 120 for culture for 5 days.

For example, when the number of cells after 5 days from seeding is 65000 cells and the number of seeded cells is 20000 cells, the doubling time (X) is log2 ÷ (log65000 - log20000) × 120 ≈ 70 hours.

### <Criteria for determining culture evaluation of cells (proliferation of cells)>

AA: The doubling time (X) is less than 24 hours.
A: The doubling time (X) is 24 hours or more and less than 28 hours.
B: The doubling time (X) is 28 hours or more and less than 36 hours.
C: The doubling time (X) is 36 hours or more.

Compositions and results are shown in Tables 1 to 5 below.

**[Table 1]**

| Peptide-conjugated polyvinyl acetal resin | | | | Resin X1 | Resin X2 | Resin X3 | Resin X4 | Resin X5 | Resin Y1 |
|---|---|---|---|---|---|---|---|---|---|
| Polyvinyl acetal moiety | | PVB | mol% | 75 | 75 | 75 | 75 | 75 | 75 |
| Graft chain | Structural unit derived from (meth) acrylate compound (D) | Acrylic acid | mol% | 22 | 24.5 | 19 | 22 | 9 | 25 |
| | Structural unit derived from (meth)acrylate compound (C) | Dodecyl acrylate | mol% | | | | | 10 | |
| Peptide moiety | GRGDS | | mol% | 3 | 0.5 | 6 | | | |
| | c-RGDfK | | mol% | | | | 3 | 6 | |
| Solubility in ethanol (maximum dissolution amount at 60°C) | | | | ○ | ○○ | Δ | Δ | ○ | ○○ |

**[Table 2]**

| Peptide-conjugated (meth) acrylic copolymer | | | | Resin X6 | Resin X7 | Resin X8 | Resin X9 |
|---|---|---|---|---|---|---|---|
| (Meth) acrylic copolymer moiety | Structural unit derived from (meth)acrylate compound (A) | Dodecyl acrylate | mol% | 70 | 70 | | |
| | | Butyl acrylate | mol% | | | 70 | |
| | | Octyl acrylate | mol% | | | | 70 |
| | Structural unit derived from (meth)acrylate compound (B) | Acrylic acid | mol% | 27 | 27 | 27 | 27 |
| Peptide moiety | GRGDS | | mol% | 3 | | 3 | 3 |
| | c-RGDfK | | mol% | | 3 | | |
| Solubility in ethanol (maximum dissolution amount at 60°C) | | | | ○○ | ○ | ○ | ○ |

**[Table 3]**

| | | | Example 1 | Example 2 | Example 3 | Example 4 | Example 5 |
|---|---|---|---|---|---|---|---|
| Coating solution | Type of resin | | Resin X1 | Resin X1 | Resin X2 | Resin X1 | Resin X3 |
| | Type of alcohol solvent | | Butanol | Butanol | Butanol | Butanol | Butanol |
| | Content of peptide-conjugated resin in 100 wt% of coating solution | wt% | 0.1 | 0.4 | 0.7 | 0.7 | 0.7 |
| | Amino acid content of peptide-conjugated resin (content ratio of peptide moiety × 5 residues) | mol% | 15 | 15 | 2.5 | 15 | 30 |
| | Amino acid content detected in coating solution | µmol/L | 142 | 566 | 165 | 991 | 1981 |
| Average thickness of cell scaffold material (resin film) | | nm | 15 | 25 | 50 | 50 | 50 |
| Culture evaluation of cells (proliferation of cells) | | | B | A | B | A | AA |

**[Table 4]**

| | | | Example 6 | Example 7 | Example 8 | Comparative Example 1 | Comparative Example 2 |
|---|---|---|---|---|---|---|---|
| Coating solution | Type of resin | | Resin X4 | Resin X5 | Resin X1 | Resin Y1 | Resin X1 |
| | Type of alcohol solvent | | Butanol | Butanol | Ethanol | Ethanol | Butanol |
| | Content of peptide-conjugated resin in 100 wt% of coating solution | wt% | 0.5 | 0.7 | 0.7 | 0.7 | 0.05 |
| | Amino acid content of peptide-conjugated resin (content ratio of peptide moiety × 5 residues) | mol% | 15 | 30 | 15 | 0 | 15 |
| | Amino acid content detected in coating solution | µmol/L | 708 | 1981 | 991 | 0 | 71 |
| Average thickness of cell scaffold material (resin film) | | nm | 30 | 50 | 50 | 50 | 10 |
| Culture evaluation of cells (proliferation of cells) | | | A | AA | A | c | C |

**[Table 5]**

| | | | Example 9 | Example 10 | Example 11 | Example 12 | Example 13 | Example 14 | Comparative Example 3 |
|---|---|---|---|---|---|---|---|---|---|
| Coating solution | Type of resin | | Resin X6 | Resin X6 | Resin X6 | Resin X7 | Resin X8 | Resin X9 | Resin X6 |
| | Type of alcohol solvent | | Butanol | Butanol | Butanol | Butanol | Butanol | Butanol | Butanol |
| | Content of peptide-conjugated resin in 100 wt% of coating solution | wt% | 0.1 | 0.5 | 1 | 1 | 1 | 1 | 0.05 |
| | Amino acid content of peptide-conjugated resin (content ratio of peptide moiety × 5 residues) | mol% | 15 | 15 | 15 | 15 | 15 | 15 | 15 |
| | Amino acid content detected in coating solution | µmol/L | 142 | 708 | 1415 | 1415 | 1415 | 1415 | 71 |
| Average thickness of cell scaffold material (resin film) | | nm | 15 | 30 | 100 | 100 | 100 | 100 | 10 |
| Culture evaluation of cells (proliferation of cells) | | | B | A | AA | AA | A | A | C |

## Claims

1. A coating solution for forming a cell scaffold, comprising:
a peptide-conjugated resin having a synthetic resin moiety and a peptide moiety; and
an alcohol solvent,
a content of the peptide-conjugated resin being 0.1 wt% or more.

2. The coating solution for forming a cell scaffold according to claim 1, wherein an amino acid content detected by a hydrolyzed amino acid composition analysis method is 75 µmol/L or more.

3. The coating solution for forming a cell scaffold according to claim 1 or 2, wherein the synthetic resin moiety has a (meth)acrylic copolymer moiety or a polyvinyl alcohol derivative moiety.

4. The coating solution for forming a cell scaffold according to any one of claims 1 to 3, wherein the peptide-conjugated resin is a peptide-conjugated (meth)acrylic copolymer having a (meth)acrylic copolymer moiety and a peptide moiety, and
the (meth)acrylic copolymer moiety has a structural unit derived from a (meth)acrylate compound (A) represented by the following Formula (A1) or the following Formula (A2): 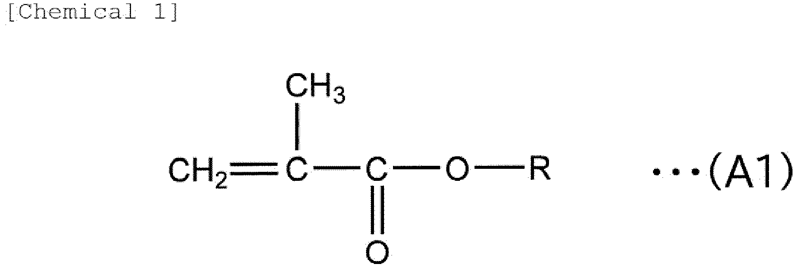
wherein R represents a hydrocarbon group having 2 or more and 18 or less carbon atoms, 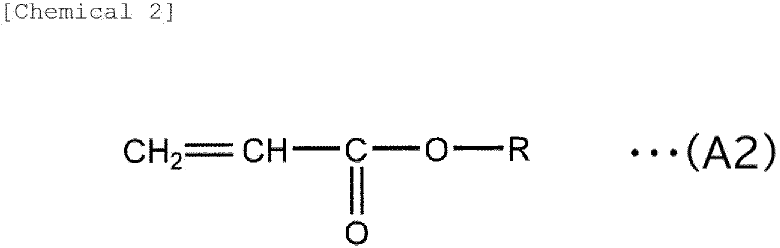
wherein R represents a hydrocarbon group having 2 or more and 18 or less carbon atoms.

5. The coating solution for forming a cell scaffold according to claim 4, wherein the (meth)acrylic copolymer moiety has a structural unit derived from a (meth)acrylate compound (B) having a functional group capable of reacting with an amino group or a carboxyl group, and
in the peptide-conjugated (meth)acrylic copolymer, the peptide moiety is bonded to the functional group capable of reacting with an amino group or a carboxyl group.

6. The coating solution for forming a cell scaffold according to any one of claims 1 to 3, wherein the peptide-conjugated resin is a peptide-conjugated polyvinyl alcohol derivative having a polyvinyl alcohol derivative moiety and a peptide moiety.

7. The coating solution for forming a cell scaffold according to claim 6, wherein the peptide-conjugated polyvinyl alcohol derivative further has a structural unit derived from a (meth)acrylate compound (C) represented by the following Formula (Cl) or the following Formula (C2): 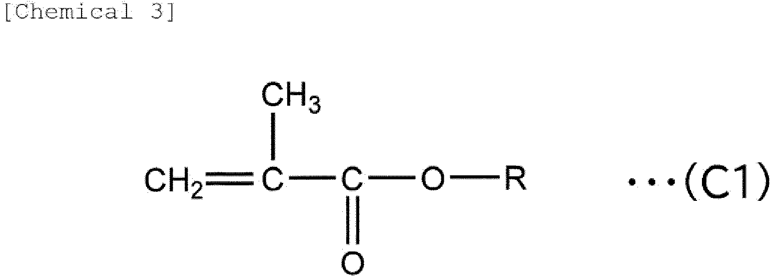
wherein R represents a hydrocarbon group having 2 or more and 18 or less carbon atoms, 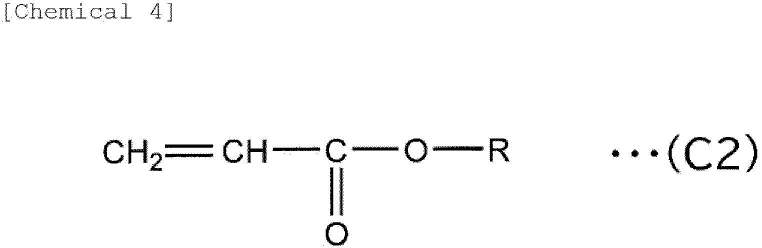
wherein R represents a hydrocarbon group having 2 or more and 18 or less carbon atoms.

8. The coating solution for forming a cell scaffold according to claim 6 or 7, wherein
the peptide-conjugated polyvinyl alcohol derivative further has a structural unit derived from a (meth)acrylate compound (D) having a functional group capable of reacting with an amino group or a carboxyl group, and
in the peptide-conjugated polyvinyl alcohol derivative, the peptide moiety is bonded to the functional group capable of reacting with an amino group or a carboxyl group.

9. The coating solution for forming a cell scaffold according to any one of claims 6 to 8, wherein the peptide-conjugated polyvinyl alcohol derivative is a peptide-conjugated polyvinyl acetal resin having a polyvinyl acetal moiety and a peptide moiety.

10. The coating solution for forming a cell scaffold according to any one of claims 1 to 9, wherein the peptide moiety has an RGD sequence.

11. A method for producing the coating solution for forming a cell scaffold according to any one of claims 1 to 10, comprising:
a preparation step of preparing a solution containing a synthetic resin having a functional group capable of reacting with an amino group or a carboxyl group, a peptide, and a condensing agent; and
a reaction step of reacting the synthetic resin with the peptide.

12. The method for producing the coating solution for forming a cell scaffold according to claim 11, further comprising a purification step.
